# EUROPEAN PATENT APPLICATION

(11) **EP 3 579 128 A1**
(43) Date of publication of application: **11.12.2019**
(21) Application number: 18176388.9
(22) Date of filing: 07.06.2018
(51) Int. Cl.: G06F 19/00, G01W 1/02

(54) **A SYSTEM AND METHOD FOR DETERMINING CONDITIONS WHICH RISK RESPIRATORY ATTACKS**

(71) Applicant: Koninklijke Philips N.V., 5656 AE Eindhoven (NL)
(72) Inventor: VAN DER SLUIS, Paul, 5656 AE Eindhoven (NL)
(74) Representative: de Haan, Poul Erik

(57) **Abstract**

A system is provided for warning of a risk of respiratory attack conditions in geographical region. Input data is obtained in the form of historical weather data, current weather data and forecast weather data, and allergen information such as pollen count data in respect of the geographical region. An advance risk warning is based on a probability of a thunderstorm and on the historical weather data. In combination, the historical weather, allergen information and probability of a thunderstorm may be used to provide a risk assessment of the occurrence of an event in which high concentrations of allergen are present. Preventative and/or preparatory measures may then be adopted.

## Description

### FIELD OF THE INVENTION

This invention relates to a system for warning a user that they are at an increased risk of suffering a respiratory attack, such as an asthma attack, so that preventative measures may be taken.

### BACKGROUND OF THE INVENTION

The spread of respiratory allergies (allergic rhinitis) is increasing. Around one third of adults in the US has respiratory allergies and up to 40% of children in the US are allergic to respiratory allergens. Pollen is the perhaps the most common, although dust mites, mold and pet dander are also allergen sources for respiratory allergies.

According to the World Health Organization (WHO) there are currently 235 million people suffering from asthma worldwide. This number is on the rise.

This invention is directed in particular to risk detection based on analysis of pollen, which is one of the main risk factors for developing this disease.

Detecting asthma triggers and symptoms is a well-studied area. Tracking symptoms and indicators in exhaled breath compounds such as 8-isoprostane, carbon monoxide (CO), and other exhaled breath biomarkers is an approach for managing asthma by means of predicting and preventing asthma attacks. This approach can also extend to monitoring the physical indicators of asthma symptoms, such as tracking wheezing sounds with an on-body acoustic detector e.g. one or more microphones.

A broader approach is to incorporate environmental data as well, such as air quality and allergen/pollen indicators, for providing a better coverage of the patient management system, as well as generating location based advice and warnings.

Thus, various approaches are known for asthma warning systems, including using breath markers, tracking environmental conditions such as allergens / pollens, giving location based advice and sharing user feedback to a networked system.

Once contracted, renewed exposure to pollen can lead to exacerbations that in some cases can even lead to death. An increasing body of evidence shows the occurrence of severe asthma epidemics during thunderstorms in the pollen season.

Such epidemic events are not only a problem for the individual patient, but are of such magnitude that they impact the health care system as a whole. A recent outbreak in Australia caused more than 8000 people to flood the hospitals, effectively paralyzing the whole healthcare system. In the end nine people died, partly due to the fact that adequate help could not be delivered due to the sudden flood of patients.

According to the Intergovernmental Panel on Climate Change (IPCC) the number of thunderstorms is expected to rise in the near future due to climate change. It is also expected that due to the warming of the climate, seasons for certain pollen will be extended. These climate events not only affect asthma patients (334 million worldwide), but also patients that suffer from allergic rhinitis (60 million in just the US).

There is therefore an increasing need to be able to predict conditions which may lead to such an asthma epidemic, so that preventative measures may be taken and/or preparations may be made for the expected surge of patients.

### SUMMARY OF THE INVENTION

The invention is defined by the claims.

According to examples in accordance with an aspect of the invention, there is provided a system for warning of a risk of respiratory attack conditions in a geographical region, comprising:
an input for receiving input data comprising historical weather data, current weather data and forecast weather data, and allergen information in respect of the geographical region; and
a processor adapted to process the received input data for providing an advance risk warning based on a probability of a thunderstorm, on the historical weather data and on the allergen information.

This system assesses a risk of conditions which cause respiratory attacks, in particular based on the combination of allergen information such as pollution levels and weather conditions, including imminent arrival of thunderstorms.

Asthma epidemics caused by severe weather events disrupt society and cause many deaths worldwide. These weather events also affect individuals with allergic rhinitis. This system provides a warning, so that preventive action be implemented. For individuals, the advice in response to a risk warning would be to seek shelter indoors just before the severe weather hits. For health care professionals the warning increases lead times and preparedness.

The risk warning may simply be a binary warning (i.e. yes or no) which is most easily understood by a user, or it may be a probability value or other analog value representing the probability of a high allergen risk, or it may be an indication of an expected allergen concentration level.

The allergen data is preferably pollen count data. It may furthermore comprise a prediction of a future pollen count. Thus, a historical pollen count and predicted pollen count may be taken into account.

The historical, current and forecast weather data for example include temperature and air moisture. These may have an influence on allergen potency.

The processor is preferably also adapted to determine a duration of a preceding period of dryness at the geographical region. If there has been a period of wet weather before a storm, the allergen potency is reduced. Thus, most potent is a period of dryness (and high pollen count) followed by a storm. Thus, the duration of a preceding period of dryness may be used to weight the thunderstorm risk value in order to arrive at a measure of allergen risk.

The advance risk warning may relate to a determined risk in a following period of a value between 1 hour and 48 hours. This is a medium term assessment, for example for the next day or two days, which is intended to give sufficient time for medical professionals to take preparatory actions.

The processor may be adapted to process the received input data for providing an imminent risk warning based the existence of a thunderstorm moving towards the geographical region and on the preceding historical weather data.

The imminent risk warning may relate to a determined risk in a following period of a value of an hour or a few hours, for example up to 2 hours. It is intended to be provided to users of the device to take immediate preventative action, for example by staying indoors.

The invention also provides a method for warning of a risk of respiratory attack conditions in geographical region, comprising:
receiving input data comprising historical weather data, current weather data and forecast weather data, and allergen information in respect of the geographical region; and
processing the received input data for providing an advance risk warning based on a probability of a thunderstorm, on the historical weather data and on the allergen information.

Thus, the risk warning is based on a high probability of a thunderstorm, high pollen levels, and also previous weather conditions which are indicative of a likely respiratory risk.

The allergen information for example comprises pollen count data and it may further comprise a prediction of a future pollen count. The historical, current and forecast weather data preferably include temperature and air moisture.

The method may comprise determining a duration of a preceding period of dryness at the geographical region and using said duration when determining if an advance risk warning is needed.

The method may comprise processing the received input data for providing an imminent risk warning based the existence of a thunderstorm moving towards the geographical region and on the preceding historical weather data.

The invention may be implemented at least in part in software, and the invention thus also includes a computer program comprising computer program code means which is adapted, when said program is run on a computer, to implement the method as defined above.

### BRIEF DESCRIPTION OF THE DRAWINGS

Examples of the invention will now be described in detail with reference to the accompanying drawings, in which:
Figure 1 shows a system for warning of a risk of respiratory attack conditions in geographical region;
Figure 2 shows a storm cloud, and is used to show how a storm progresses;
Figure 3 shows a rain radar map of a storm;
Figure 4 shows a method for warning of a risk of respiratory attack conditions in geographical region; and
Figure 5 illustrates an example of a computer for implementing the processor.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for warning of a risk of respiratory attack conditions in geographical region. Input data is obtained in the form of historical weather data, current weather data and forecast weather data, and allergen information such as pollen count data in respect of the geographical region. An advance risk warning is based on a probability of a thunderstorm and on the historical weather data. In combination, the historical weather, allergen information and probability of a thunderstorm may be used to provide a risk assessment of the occurrence of an event in which high concentrations of allergen are present. Preventative and/or preparatory measures may then be adopted.

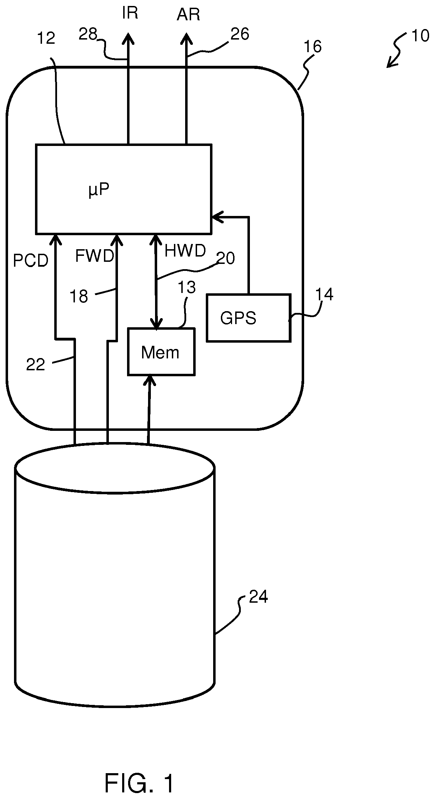

Figure 1 shows a system 10 in accordance with one example of the invention. The system shown is associated with (and carried by) a particular user.

The system 10 is for warning of a risk of respiratory attack conditions in geographical region.

It comprises a processor 12 and a location indicator 14 for identifying or tracking the location of the user. The location indicator is for example a location sensor such as a GPS sensor. However, other methods of identifying location may be used, such as imaging information, for example images from Google Glass. These images may also enable location to be tracked over the time period of interest.

A memory 13 is used for storing data over a moving time window, so that historical data is available when needed.

The processor and location indicator may be part of a mobile telephone or tablet 16. Thus, the invention may be implemented by loading suitable software onto a mobile telephone, which may already include all the hardware necessary to implement the system of the invention.

The processor has a first input 18 for receiving forecast weather data ("FWD") and a second input 20 for receiving historical weather data ("HWD") for a preceding time period. This data is received from the memory 13. The historical weather data may be considered to include the current weather information, so that the processor also receives the current weather data (e.g. as the most up to date historical weather data).

The processor has a third input 22 for receiving allergen information, in particular pollen count data ("PCD"). The allergen information may however include other information such as one or more particulate concentration values.

The weather data and allergen information is obtained from a database 24, for example by connection over the internet. The data is for example hosted by a weather station.

The processor 12 generates an output in the form of an advance risk warning ("AR") based on a probability of a thunderstorm and on the historical weather data.

In the example shown there are two type of risk warning. One output 26 is a longer term advance risk warning ("AR") and another output 28 is an imminent risk warning ("IR").

The system assesses a risk of conditions which cause respiratory attacks, in particular based on the combination of pollution levels and weather conditions, including imminent arrival of thunderstorms.

This system provides a warning, so that preventive action be implemented. For individuals, the advice in response to a risk warning would be to seek shelter indoors just before the severe weather hits. For health care professionals the warning increases lead times and preparedness.

For individuals, the geographical region which is processed by the system may be a small area in the vicinity of the user. For health care professionals - e.g. forming a public health warning system - the geographical region which is processed by the system may be a large region, for example in which multiple weather fronts and different local weather conditions are monitored.

The risk warning may simply be a binary warning (i.e. yes or no) which is most easily understood by a user, or it may be a probability value or other analog value representing the probability of a high allergen risk, or an allergen risk level. The risk warning may relate to a single location in the vicinity of the user, or it may be a map of different locations and associated risks when the system is for providing risk assessment in respect of a wider geographical region.

For a thunderstorm-induced asthma event to occur, several conditions have to be met. The aim of the system is to identify those conditions, in order to coach health care services and patients.

First of all the allergens have to be present. This is not only the pollens themselves but also ruptured pollens and possibly also other plant parts such as orbicules. Thus, the allergen information may relate to pollen and/or other plant parts and even other pollutants. Pollen counts and predictions of pollen counts can be used as a proxy for the general allergen potency of the atmosphere.

In addition, areas that received recent rainfall have less potential to create airborne particles, simply because the soil is wet. Thus, a map of allergen potency may be derived from the current (and predicted) allergen information (e.g. pollen count information), but the significance of this may be adapted by taking into account the historical weather conditions.

By way of example, a map of allergen potency may be weighted with a parameter which depends on the number of preceding dry days.

The parameter maybe a multiplier. Thus, in one example a pollen count may be converted to a risk-weighted pollen count by multiplication with *nk* where *n* is the number of preceding dry days and *k* is a multiplier to make the multiplication appropriate. This may be obtained based on previous experimentation.

Further refinement can be carried out based on temperature and air moisture. This weather data is also available from weather stations. Thus, a risk-weighted pollen count may be obtained which takes account of actual pollen count levels and current and preceding weather conditions.

This risk-weighted pollen count is then a measure of the risk that severe allergen conditions will arise if there is a thunderstorm (or a storm more generally). Thus, by multiplying the risk measure by the probability of a thunderstorm, an overall risk assessment is obtained. This overall risk is a probability that a thunderstorm takes place with the prediction time window and that the pollen and preceding weather conditions are such that it will result in a high pollen exposure, e.g. passing a pollen concentration threshold which represents a severe risk.

As mentioned above, the eventual output may be a simply binary indicator. However, the output information may instead separately indicate the risk of a storm and the pollen conditions.

The potential for the development of thunderstorms will give an advance warning typically of one day. This warning is available from weather services and is based on atmosphere instability. Such a warning is not very specific; it generally only gives a probability of a thunderstorm developing in a certain area.

However, combined with the risk-weighted allergen potency map as explained above, a more reliable advance warning system is made available for health care services. This provides a readiness alert for events that may happen in the next 24 hours, and is the longer term advance warning.

The imminent warning is for users of the system (or other people to be warned by the users of the system) who are at more imminent risk. A short notice (e.g. less than 1 hour) warning may be provided but with a higher accuracy. Allergen sufferers should then move indoors with the windows closed until the event has passed.

As explained above, one factor is the assessment of the probability of a thunderstorm (or any storm, more generally).

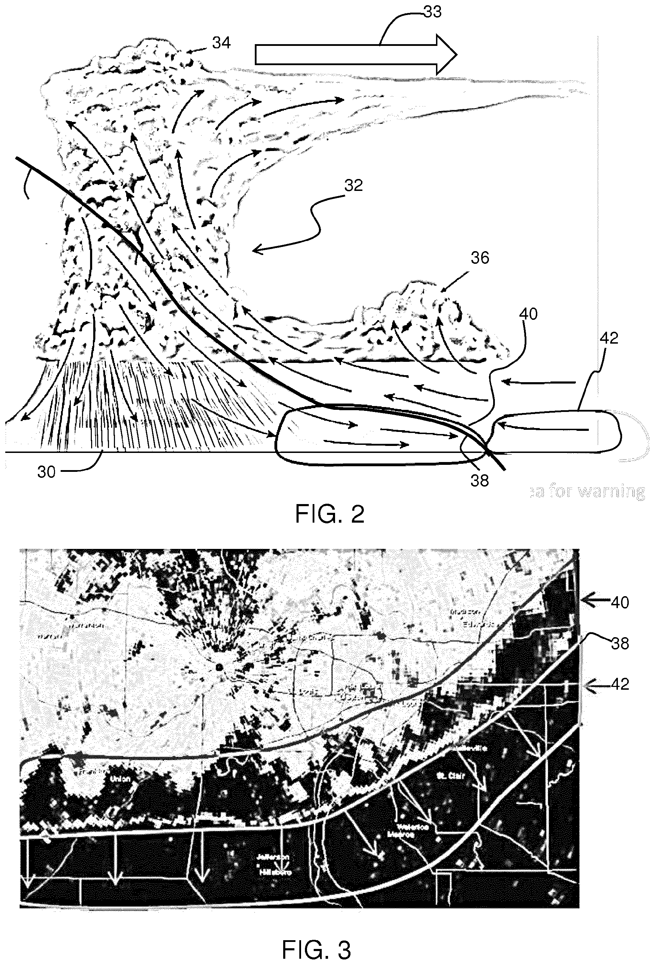

Figure 2 shows a cross section of a storm, showing the ground 30, and the cloud cover 32. The storm is advancing in the direction of arrow 33. The cloud cover has a overshooting top 34 and a developing cell 36. The storm generates heavy rain and a cold downdraft. The storm defines a gust front 38.

The gust front 38 shows up in a rain radar image in front of the storm as a thin line. This can be seen in Figure 3. In a rain radar image, storms can be identified by the occurrence of a high reflectivity. However, key for the allergen event to occur is not the occurrence of a high reflectivity or the presence of lightning, but a strong gust front caused by the low level outflow of the storm.

It is this front of high winds that causes the allergic particles to become airborne in high amounts which in turn causes the severe reactions observed in patients. Thus, the monitoring of storms in particular involves detecting the location and movement of the gust fronts associated with the storms.

Of course, the advance warning is in respect of the area in front of the storm, indicated by the warning zone area 42 in Figures 2 and 3. The area 40 behind the gust front 38 is where the greatest danger lies. Shortly after the heavy rains start, the danger levels drop because the air is cleared by the rain.

A warning only needs to be given when the risk-modified allergen map shows a high risk in the warning zone 42. This prevents warnings for example in wet conditions when no allergens can be swept into the air or warnings in winter when allergens are not present.

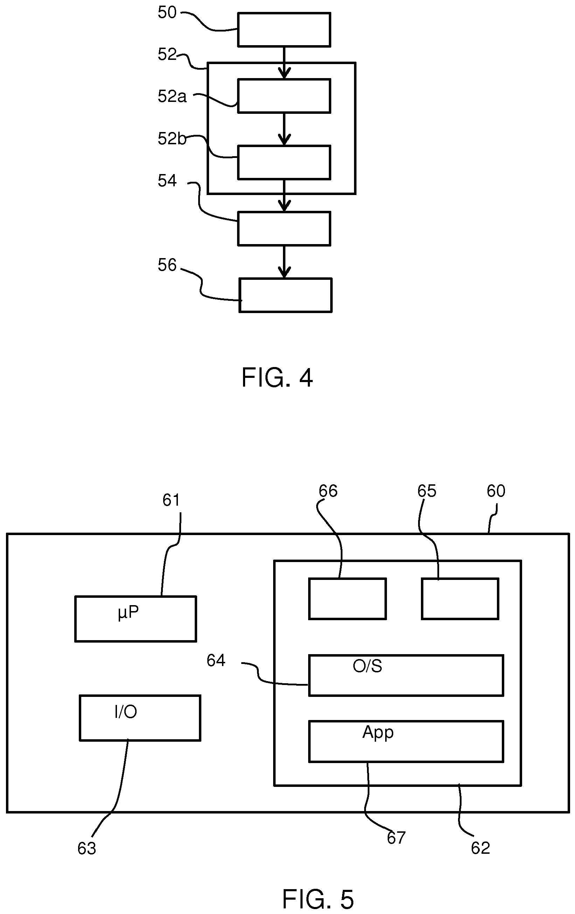

Figure 4 shows a method for warning of a risk of respiratory attack conditions in geographical region, comprising:
in step 50, receiving input data comprising historical weather data, current weather data and forecast weather data, and allergen information, such as pollen count data, in respect of the geographical region;
in step 52 processing the received input data; and
in step 54 providing an advance risk warning based on a probability of a thunderstorm and on the historical weather data.

Thus, the risk warning is based on a high probability of a thunderstorm, high pollen levels, and also previous weather conditions which are indicative of a likely respiratory risk.

The processing 52 for example comprises in step 52a determining a duration of a preceding period of dryness at the geographical region and in step 52b using said duration when determining if an advance risk warning is needed, in particular by converting a pollen count to a risk-weighted pollen count.

The method also comprises providing an imminent risk warning in step 56 based on the existence of a thunderstorm moving towards the geographical region and on the preceding historical weather data.

The system described above makes use of a processor 12 for processing data.

Figure 5 illustrates an example of a computer 60 for implementing the processor described above.

The computer 60 includes, but is not limited to, PCs, workstations, laptops, PDAs, palm devices, servers, storages, and the like. Generally, in terms of hardware architecture, the computer 60 may include one or more processors 61, memory 62, and one or more I/O devices 63 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 61 is a hardware device for executing software that can be stored in the memory 62. The processor 61 can be virtually any custom made or commercially available processor, a central processing unit (CPU), a digital signal processor (DSP), or an auxiliary processor among several processors associated with the computer 60, and the processor 61 may be a semiconductor based microprocessor (in the form of a microchip) or a microprocessor.

The memory 62 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 62 may incorporate electronic, magnetic, optical, and/or other types of storage media. Note that the memory 62 can have a distributed architecture, where various components are situated remote from one another, but can be accessed by the processor 61.

The software in the memory 62 may include one or more separate programs, each of which comprises an ordered listing of executable instructions for implementing logical functions. The software in the memory 62 includes a suitable operating system (O/S) 64, compiler 65, source code 66, and one or more applications 67 in accordance with exemplary embodiments.

The application 67 comprises numerous functional components such as computational units, logic, functional units, processes, operations, virtual entities, and/or modules.

The operating system 64 controls the execution of computer programs, and provides scheduling, input-output control, file and data management, memory management, and communication control and related services.

Application 67 may be a source program, executable program (object code), script, or any other entity comprising a set of instructions to be performed. When a source program, then the program is usually translated via a compiler (such as the compiler 65), assembler, interpreter, or the like, which may or may not be included within the memory 62, so as to operate properly in connection with the operating system 64. Furthermore, the application 67 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 63 may include input devices such as, for example but not limited to, a mouse, keyboard, scanner, microphone, camera, etc. Furthermore, the I/O devices 63 may also include output devices, for example but not limited to a printer, display, etc. Finally, the I/O devices 63 may further include devices that communicate both inputs and outputs, for instance but not limited to, a network interface controller (NIC) or modulator/demodulator (for accessing remote devices, other files, devices, systems, or a network), a radio frequency (RF) or other transceiver, a telephonic interface, a bridge, a router, etc. The I/O devices 63 also include components for communicating over various networks, such as the Internet or intranet.

When the computer 60 is in operation, the processor 61 is configured to execute software stored within the memory 62, to communicate data to and from the memory 62, and to generally control operations of the computer 60 pursuant to the software. The application 67 and the operating system 64 are read, in whole or in part, by the processor 61, perhaps buffered within the processor 61, and then executed.

When the application 67 is implemented in software it should be noted that the application 67 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The monitor may be for use by an individual or by a medical establishment. Individuals may instead be warned by the medical establishment or other centralized system manager. For example, centrally the conditions for a wide geographical area may be monitored. Messages may then be sent to subscribers to the system when there are conditions within that wide geographical area where there are high risks. This may take the form of a risk map, and the user then compares their location with the risk map. This could of course be an automatic process, using a smart phone with GPS. The user is then presented with a warning if they are in a danger zone as identified by the central system manager.

Thus, the invention may be part of a public health warning system.

The system of Figure 1 includes a location sensor. However, if forming part of a centralized system, it is not needed, since information for a whole geographical region is collected and processed. Only the individual users then need to application location information to generate a user-specific warning.

A system for individual users may incorporate other health monitoring system. It may provide an assessment of the risk of that particular user having an allergen attack by taking additional factors into account, such as activity level information about the physical activity level of the user, a heart rate of the user, a breathing count as well as information about medication taken by the user.

The invention is of interest for asthma patient monitoring systems, public health management systems, allergy management systems, air quality monitoring networks, and respiratory track conditions management.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

## Claims

1. A system (10) for warning of a risk of respiratory attack conditions in a geographical region, comprising:
an input (18, 20, 22) for receiving input data comprising historical weather data (HWD), current weather data and forecast weather data (FWD), and allergen data (PCD) in respect of the geographical region; and
a processor (12) adapted to process the received input data for providing an advance risk warning (AR) based on a probability of a thunderstorm, on the historical weather data and on the allergen information.

2. A system as claimed in claim 1, wherein the allergen data comprises pollen count data (PCD) and further comprises a prediction of future pollen count.

3. A system as claimed in claim 1 or 2, wherein the historical, current and forecast weather data include temperature and air moisture levels.

4. A system as claimed in any one of claims 1 to 3, wherein the processor (12) is adapted to determine a duration of a preceding period of dryness at the geographical region.

5. A system as claimed in any one of claims 1 to 4, wherein the advance risk warning (AR) relates to a determined risk in a following period of between 1 hour and 48 hours.

6. A system as claimed in claim 5, wherein the advance risk warning (AR) relates to a determined risk in a following period of 24 hours.

7. A system as claimed in any one of claims 1 to 6, wherein the processor (12) is adapted to process the received input data for providing an imminent risk warning (IR) based the existence of a thunderstorm moving towards the geographical region and on the preceding historical weather data.

8. A system as claimed in claim 7, wherein the imminent risk warning (IR) relates to a determined risk in a following period of up to 2 hours.

9. A system as claimed in claim 8, wherein the imminent risk warning (IR) relates to a determined risk in a following period of 1 hour.

10. A method for warning of a risk of respiratory attack conditions in geographical region, comprising:
(50) receiving input data comprising historical weather data (HWD), current weather data and forecast weather data (FWD), and allergen information (PCD) in respect of the geographical region;
(52) processing the received input data; and
(54) providing an advance risk warning (AR) based on a probability of a thunderstorm, on the historical weather data (HWD) and on the allergen information.

11. A method as claimed in claim 10, wherein the allergen information comprises pollen count data (PCD) and further comprises a prediction of a future pollen count, and wherein the historical, current and forecast weather data include temperature and air moisture levels.

12. A method as claimed in claims 10 or 11, wherein the processing the receiving input data comprises (52a) determining a duration of a preceding period of dryness at the geographical region and (54b) using said duration when determining if an advance risk warning is needed.

13. A method as claimed in any one of claims 10 to 12, comprising (56) processing the received input data for providing an imminent risk warning based the existence of a thunderstorm moving towards the geographical region and on the preceding historical weather data.

14. A method as claimed in claim 13, wherein the advance risk warning relates to a determined risk in a following period of between 1 hour and 48 hours and the imminent risk warning relates to a determined risk in a following period of up to 2 hours.

15. A computer program comprising computer program code means which is adapted, when said program is run on a computer, to implement the method of any one of claims 10 to 14.
